# EUROPÄISCHE PATENTSCHRIFT

(11) **EP 0 763 601 B1**
(45) Veröffentlichungstag und Bekanntmachung des Hinweises auf die Patenterteilung: **02.01.2008**
(21) Anmeldenummer: 96113136.4
(22) Anmeldetag: 16.08.1996
(51) Int. Cl.: C12Q 1/68

(54) **Homogener Gensondentest mit einem gegen das Label gerichteten Rezeptor**
Homogeneous hybridization assay with label specific receptors
Test d'hybridation homogène avec un récepteur pour le marquer

(30) Priorität: 14.09.1995 DE 19534122
(43) Veröffentlichungstag der Anmeldung: 19.03.1997
(73) Patentinhaber: Dade Behring Marburg GmbH, 35001 Marburg (DE)
(72) Erfinder: Neuenhofer, Stephan, Dr., 35037 Marburg (DE); Skrzipczyk, Heinz Jürgen, Dr., 63263 Zeppelinheim (DE); Madry, Norbert, Dr., 35041 Marburg (DE); Leutsch, Thomas, 35041 Marburg (DE); Käsmarker, Reinhard, 35041 Marburg (DE); Uhlmann, Eugen, Dr., 61479 Glashütten (DE)

(56) Entgegenhaltungen:
- EP-A- 0 144 913
- EP-A- 0 492 570
- WO-A-94/03643

## Beschreibung

Die Erfindung betrifft einen homogenen Gensondentest, der darauf beruht, daß das Signal der nicht hybridisierten Gensonde durch einen gegen das Label gerichteten Rezeptor verändert wird.

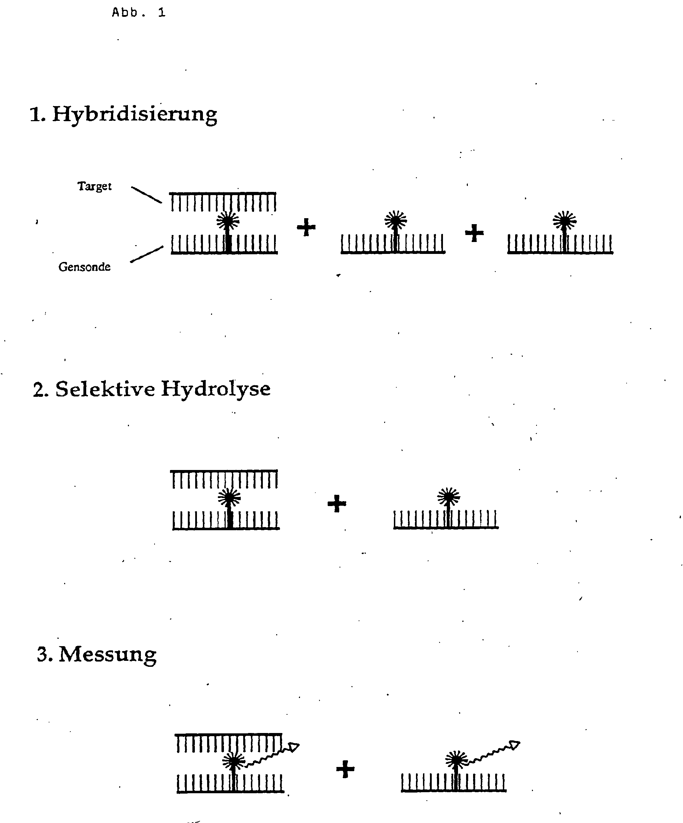

Gensondenassays sind in der Literatur in verschiedenen Ausführungsformen bereits beschrieben. Häufiger verwandte Ausführungsformen sind der "hybridization protection assay" (Clin. Chem. 35/8, 1989, 1588-1594), die Technik der "Kissing Probes" (Nachr. Chem. Tech. Lab. 37/7, 1989, 698) und das "Energy transfer"-Prinzip . Ein sehr allgemeines Prinzip eines Gensondenassays gemäß dem Stand der Technik ist in Abb. 1 wiedergegeben: im ersten Schritt werden Targetsequenz und markierte Gensonde miteinander hybridisiert, woraus bei hinreichender Homologie beider Sequenzen doppelsträngige Konstrukte resultieren. Darüberhinaus bleiben in der Regel aber auch nicht hybridisierte einzelsträngige Anteile der Gensonde übrig. Im zweiten Schritt wird eine selektive Hydrolyse durchgeführt, welche dadurch charakterisiert ist, daß aufgrund der gewählten Bedingungen im wesentlichen das Label der einzelsträngigen Gensonde angegriffen wird, während das Label des doppelsträngigen Konstrukts vor einem hydrophilen Angriff weitgehend geschützt ist. Somit wird dann im dritten Schritt im wesentlichen das durch das im doppelsträngigen Konstrukt gebundene Label hervorgerufene Signal gemessen.

Ein Nachteil obigen Verfahrens besteht darin, daß trotz der Behandlung mit dem Selektionsreagenz (Schritt 2) ein Rest einzelsträngiger Gensonde mit intaktem Label zurückbleibt, welches die Messung verfälscht.

Der vorliegenden Erfindung liegt deshalb die Aufgabe zugrunde, ein Verfahren zur Bestimmung einer Nukleinsäuresequenz (einen "Gensondenassay") zur Verzufügung zu stellen, in welchem die nicht hybridisierte markierte Gensonde in einem geringeren Ausmaß zu einer unerwünschten Signalbildung beiträgt, als in den Verfahren gemäß dem Stand der Technik. Die angestrebte Verbesserung soll insbesondere eine verbesserte homogene Testausführung ermöglichen. Die homogene Testausführung ist grundsätzlich durch das Fehlen eines physikalischen Trennschrittes zwischen der Nukleinsäurehybridisierung und dem Signalnachweis charakterisiert. In einem solchen Verfahren ist gemäß dem Stand der Technik mit einem besonders starken Störeinfluß durch die nicht hybridisierte markierte Gensonde zu rechnen.

EP0144913 offenbart ein Verfahren in dem ein "anti-fluorescer antibody" und ein "anti-hybrid antibody" zum Einsatz kommen um der Störeinfluss der nicht hybridisierten Gensonden zu reduzieren.

EP0492570 offenbart eine chemische Modifikation des Labels um der Störeinfluss der nicht hybridisierten Gensonden zu reduzieren.

Die Aufgabe wurde überraschenderweise dadurch gelöst, daß in dem erfindungsgemäßen Verfahren ein Rezeptor eingesetzt wird, der an das Label binden kann und als Folge der Bindung das auf das Label zurückzuführende Signal detektierbar verändert, beispielsweise abschwächt ("quencht"). Durch das nachfolgend beschriebene erfindungsgemäße Verfahren ist es daher möglich, den Störeinfluß durch die nicht hybridisierte markierte Gensonde signifikant zu reduzieren und dadurch die Sensitivität und Spezifität des Testsystems entscheidend zu verbessern.

Die vorliegende Erfindung betrifft somit ein Verfahren zur Bestimmung einer Nukleinsäuresequenz (=Targetsequenz), worin eine die Targetsequenz gegebenenfalls enthaltende Probe mit einer zur Bestimmung dieser Targetsequenz geeigneten Gensonde so in Kontakt gebracht wird, daß die Targetsequenz und die Gensonde miteinander hybridisieren, dadurch gekennzeichnet, daß außerdem
(a) ein Rezeptor zugesetzt wird, der an das Label einer gegebenenfalls überschüssigen, nicht an die Targetsequenz hybridisierten Fraktion der Gensonde bindet, wodurch das auf das Label zurückzuführende Signal qualitativ und/oder quantitativ verändert wird und
(b) das auf das Label zurückzuführende Signal mit einem dafür geeigneten Verfahren qualitativ oder quantitativ detektiert wird.

Die vorliegende Erfindung betrifft weiterhin ein Verfahren, worin in einem zusätzlichen Schritt das Label einer gegebenenfalls überschüssigen, nicht hybridisierten Gensonde durch Inkubation mit einem Selektionsreagenz teilweise inaktiviert wird.

Eine bevorzugte Ausgestaltungsform vorliegender Erfindung ist dadurch gekennzeichnet, daß zuerst die Inkubation mit dem Selektionsreagenz und anschließend die Zugabe des Rezeptors erfolgt.

Weiterhin bevorzugt ist ein Verfahren, in dem der Rezeptor ein monoklonaler oder polyklonaler Antikörper, ein Antikörperfragment, ein chemisch modifizierter Antikörper oder ein chemisch modifiziertes Antikörperfragment ist, sofern die Antigenbindungsfähigkeit nach der chemischen Modifikation in ausreichendem Maße erhalten bleibt.

Weiterhin bevorzugte erfindungsgemäße Verfahren sind dadurch gekennzeichnet, daß das Label eine zur Fluoreszenz, Phosphoreszenz, Chemilumineszenz, Biolumineszenz oder Elektrolumineszenz befähigte Gruppe ist.

In einer besonders bevorzugten Ausgestaltungsform vorliegender Erfindung ist das Label ein Acridiniumester, ein Acridiniumacylsulfonamid, ein Luminol, ein Isoluminol oder ein Derivat davon, ein Dioxetan, ein Luciferin, ein Oxalsäureester oder ein Oxalsäureamid.

Ebenfalls bevorzugt ist ein Verfahren, in welchem das Label ein Enzym ist.

### Label:

Als Label geeignet sind alle zur Fluoreszenz, Phosphoreszenz, Chemilumineszenz, Biolumineszenz oder Elektrolumineszenz befähigten Gruppen, die aufgrund ihrer chemischen Struktur in einer solchen Weise mit einem Nucleinsäuredoppelstrang in Wechselwirkung treten können, beispielsweise durch Interkalieren in den Doppelstrang, daß die Bindung eines gegen diese Gruppe gerichteten Rezeptors im Vergleich zur Bindung an die entsprechende einzelstranggebundene Gruppe erschwert wird. Besonders geeignet sind Acridiniumester- und Acridiniumacylsulfonamid-Gruppen, die in eine doppelsträngige Nukleinsäure interkalieren. Außerdem geeignet ist ein Luminol, ein Isoluminol oder ein Derivat davon, ein Dioxetan, ein Luciferin, ein Oxalsäureester oder ein Oxalsäureamid.

Lumineszierende Verbindungen finden bereits vielfältige Anwendung. Sie werden als Indikatoren in Enzymimmunoassays, Lumineszenzimmunoassays (vgl. W.P. Collins "Alternative Immunoassays", Verlag John Wiley & Sons Ltd., Chichester, 1985) und Bioassays (Teste, die nicht auf Antigen-Antikörperwechselwirkungen, sondern auf Bindungsaffinitäten zwischen Molekülen, die nicht dem Immunsystem zugerechnet werden beruhen), aber auch in Nukleinsäurehybridisierungsassays eingesetzt (vgl. J.A. Matthews et al. "Analytical Biochemistry", 151, 205-209, 1985). Außerdem werden chemilumineszierende Verbindungen bei der "flow injection analysis", in "post-column"-Detektoren in der Flüssigkeitschromatographie, in der Strömungsforschung sowie zur Herstellung künstlicher Lichtquellen verwendet. Acridinderivate eignen sich weiterhin in Testverfahren zur Lebensmittel- und Umweltanalyse.

Die Verwendung von Acridiniumlabeln in Nukleinsäurehybridisierungsassays wird in der EP-A-0 273 115 erwähnt sowie in der EP-A-0 212 951, der EP-A-0 281 390, der EP-A-0 310 312, der EP-A-0 313 219 und der WO 89/02896 beschrieben. In der EP-A-0 407 816 werden Nucleotid-Derivate mit der Base Uracil beschrieben, die ihrerseits über einen Spacer mit einer chemilumineszierenden Verbindung markiert ist. In der EP A 602 524 werden lumineszenzmarkierte Gensonden mit gegenüber dem Stand der Technik vorteilhaften Eigenschaften sowie u.a. ein homogener Gensondenassay nach dem "hybridization protection assay"-Prinzip, der auf den vorteilhaften Eigenschaften der offenbarten Gensonden aufbaut beschrieben.

### Anti-Label-Antikörper:

Gegen das Label gerichtete Antikörper lassen sich grundsätzlich auf konventionelle Weise, z.B. durch Immunisieren eines Versuchstieres mit dem Label und anschließende Selektion geeigneter signalbeeinflussender Antikörper herstellen. Geeignet sind sowohl polyklonale, als auch monoklonale Antikörper, wobei monoklonale Antikörper (MAK) bevorzugt werden. Einige gegen luminogene Acridiniumlabel gerichtete Antikörper besitzen die Eigenschaft, durch Bindung des Labels dessen Signalstärke zu reduzieren (Quencheffekt). So wurde beispielsweise in einem einzigen Versuchsansatz unter 10 gegen ein luminogenes Acridiniumacylsulfonamidlabel gerichteten Maus-MAK, welche hinsichtlich möglicher signalquenchender Eigenschaften nicht vorselektiert waren, ein MAK gefunden, welcher die gewünschten signalquenchenden Eigenschaften besaß.

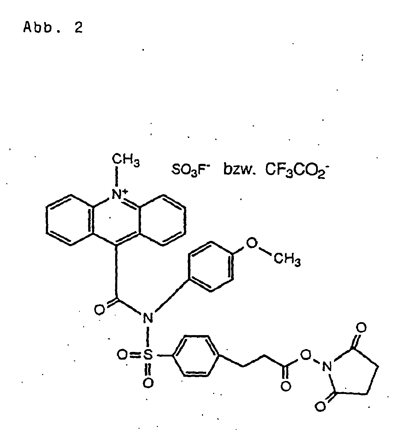

Ein Beispiel für einen gut geeigneten Antikörper ist der aus der Zellinie BW 90-614-8-04 sezernierte monoklonale Maus-Antikörper, welche bei der Deutschen Sammlung von Mikroorganismen und Zellkulturen GmbH, Mascheroder Weg 1B, D-38124 Braunschweig unter der Eingangsnummer DSM ACC 2184 hinterlegt wurde. Dieser MAK ist gegen das in Abb. 2 gezeigte Acridiniumacylsulfonamid gerichtet.

### Herstellung der Gensonden:

Die Herstellung geeigneter Gensonden kann mit dem Fachmann prinzipiell bekannten Methoden vorgenommen werden. In einer größeren Anzahl von Veröffentlichungen werden Gensonden ausführlich besprochen, beispielsweise in: S. L. Beaucage and R. P. Iyer: "The Functionalization of Oligonucleotides Via Phosphoramidite Derivatives"; Tetrahedron 49, 1925-1963 (1993) und J. Goodchild: "Conjugates of Oligonucleotides and Modified Oligonucleotides: A Review of Their Synthesis and Properties"; Bioconjugate Chemistry 1, 165-187 (1990).

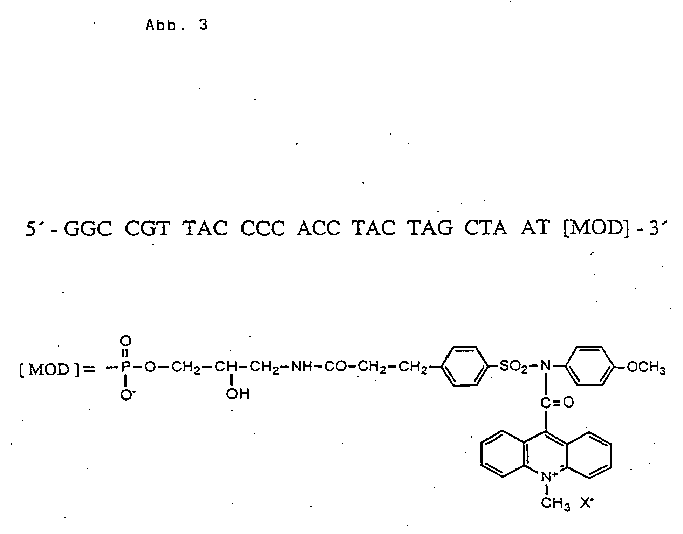

Die Struktur einer geeigneten Gensonde ist beispielhaft in Abb. 3 dargestellt. Selbstverständlich kann die gezeigte Basensequenz durch jede andere geeignete Sequenz ersetzt werden. Ebenso können andere jedem Fachmann bekannte Label nach jedem Fachmann bekannten Verfahren mit der als Gensonde zu verwendenden Nukleinsäure verknüpft werden.

### Der Gensondenassay:

Generell läßt sich das erfindungsgemäße Verfahren auf Basis aller im Stand der Technik bekannten Verfahren verwirklichen. Besonders vorteilhaft läßt sich die erfindungsgemäße Gensondentechnologie in homogenen Testen einsetzen. Aufgrund des starken Signalquenches durch einen Anti-Label-MAK lassen sich bei vergleichbar guter Stabilität wesentlich sensitivere homogene Gensondenteste entwickeln, als sie im Stand der Technik bekannt sind. Erfindungsgemäße homogene Gensondenassays zeichnen sich außerdem durch einfache Handhabung und leichte Automatisierbarkeit aus.

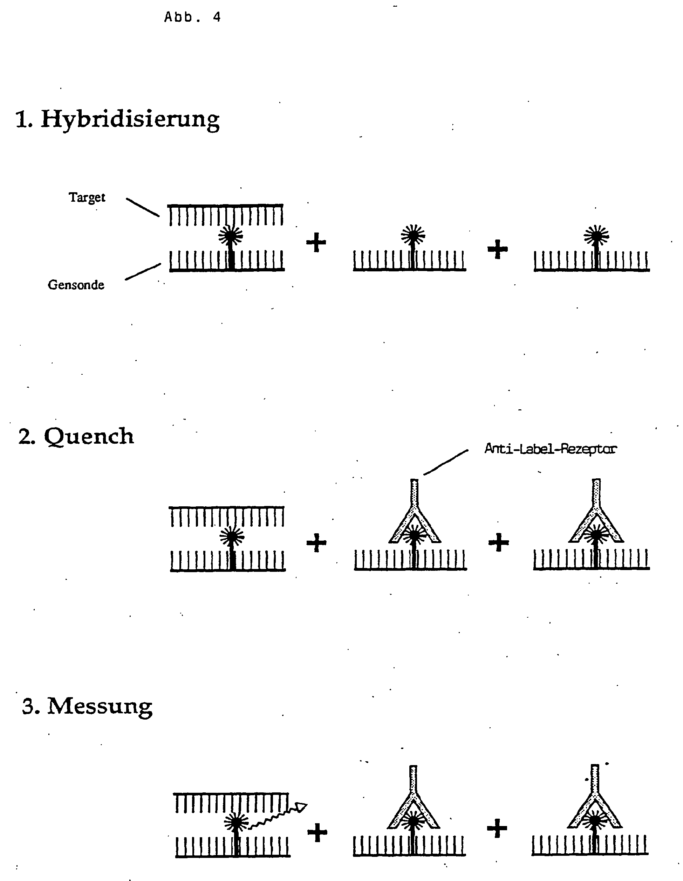

Eine bevorzugte Variante des erfindungsgemäßen Verfahrens ist schematisch in Abb. 4 dargestellt: im ersten Schritt werden Targetsequenz und markierte Gensonde miteinander hybridisiert, woraus bei hinreichender Homologie beider Sequenzen doppelsträngige Konstrukte resultieren. Darüberhinaus verbleiben in der Regel auch nicht hybridisierte einzelsträngige Anteile der Gensonde übrig. Im zweiten Schritt wird ein Rezeptor, beispielsweise ein Antikörper, eingesetzt, der an das Label der einzelsträngigen Gensonde binden kann und als Folge der Bindung das auf das Label zurückzuführende Signal detektierbar verändert, beispielsweise abschwächt (quencht). Im dritten Schritt wird dann fast ausschließlich das durch das im doppelsträngigen Konstrukt gebundene Label hervorgerufene Signal gemessen, da das Label der hybridisierten Gensonde nicht oder weniger gut vom Rezeptor gebunden werden kann, als das Label der nicht hybridisierten Gensonde. Dieses Verfahren hat gegenüber bekannten Verfahren den Vorteil, daß noch weniger einzelsträngige Gensonde einen (unerwünschten) Beitrag zum Meßsignal liefert.

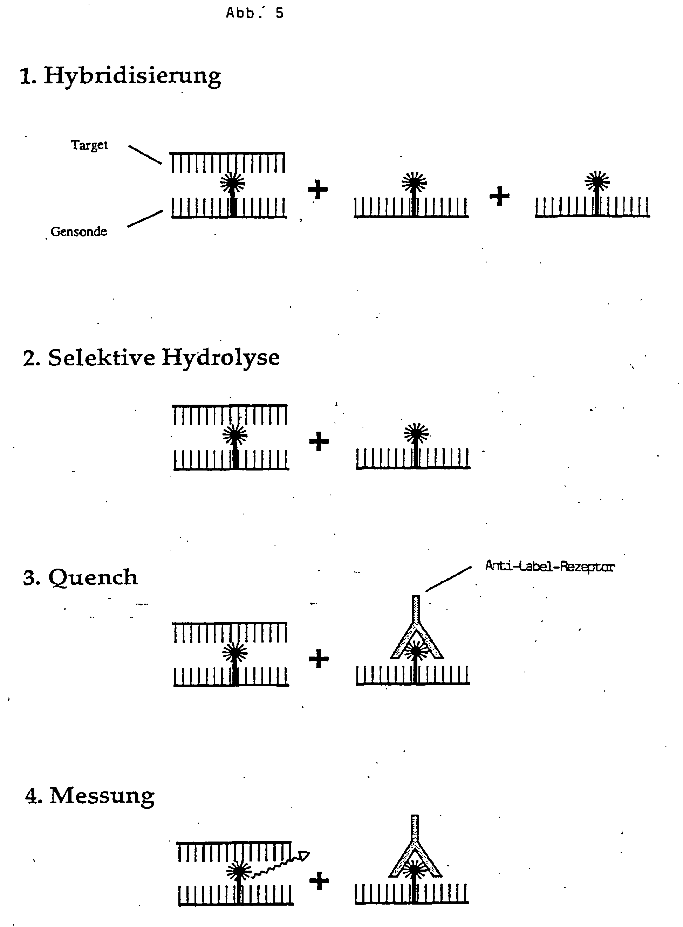

Obiges Verfahren kann dadurch weiter verbessert werden, daß nach dem ersten Hybridisierungsschritt durch Behandlung mit einem Selektionsreagenz eine selektive Hydrolyse der einzelsträngigen Gensonde vorgenommen wird und erst daran anschließend ein gegen das Label gerichteter Rezeptor eingesetzt wird, wie oben dargestellt. Die sich nun anschließende Messung wird durch die vorangegangene zweifache Elimination des Labels der einzelsträngigen Gensonde praktisch nicht mehr durch ungebundenes Label verfälscht (s. Abb. 5).

Eine weitere bevorzugte Ausführungsvariante basiert auf dem "hybridization protection assay" (s.o.) und ist erfindungsgemäß dadurch gekennzeichnet, daß das Label der nicht hybridisierten einzelsträngigen Anteile der Gensonde in einem weiteren Schritt durch Zusatz eines Rezeptors, der an das Label der einzelsträngigen Gensonde binden kann, detektierbar verändert, z.B. gequencht wird, so daß diese ungebundenen Gensonden keine Meßsignalverfälschung bewirken können.

Als Rezeptor wird in dem erfindungsgemäßen Verfahren bevorzugt ein gegen das Label gerichteter, siglalquenchender monoklonaler oder polyklonaler Antikörper eingesetzt, wie bereits weiter oben beschrieben.

Die nachfolgendenden Beispiele sollen die vorliegende Erfindung weiter erläutern, diese jedoch in keiner Weise einschränken.

### Beispiel 1: Herstellung eines monoklonalen Antikörpers gegen ein luminogenes Acridiniumacylsulfonamid-Label:

Für die Herstellung monoklonaler Antikörper wurde BALB-c-Mäusen subkutan oder intraperitoneal 10 µg Acridiniumacylsulfonamid-BSA-Konjugat, emulgiert in komplettem Freund'schen Adjuvans injiziert. Das Acridiniumacylsulfonamid-BSA-Konjugat ist durch Umsetzung von N-(4-Methoxyphenyl)-N-[4-(2-succinimidyloxycabonylethyl)benzolsulfonyl]-10-methylacridinium-9-carbonsäureamidfluorsulfonat oder -trifluoracetat (Abb. 2) mit BSA nach dem Fachmann bekannten Verfahren herstellbar. Es folgten 4 bis 5 zusätzliche Immunisierungen ohne Adjuvans alle vier Wochen. Die letzten vier Tage vor der Fusion erhielten die Mäuse intravenöse Auffrischungsinjektionen (10 µg pro Tag).

Zur Herstellung von Hybridomen wurden die immunisierten Tiere mittels zervikaler Luxation getötet. Die Milz wurde aseptisch entfernt und auseinandergezupft, um eine Einzelsuspension von Milzzellen in serumfreiem nach Dulbecco modifiziertem Eagle Medium (DMEM) zu erhalten. Die Zellen wurden mittels Zentrifugation (5 Min.; 1800 Upm.) gesammelt und einmal in DMEM gewaschen. Die Gesamtzellenanzahl wurde durch Hämocytometer-Zählung unter Verwendung der Trypanblau-Exklusionstechnik bestimmt. Die Myelomzellen der Maus (SP2/0) wurden zweimal in serumfreiem DMEM gewaschen, mittels Zentrifugation gesammelt (10 Min., 1000 Upm.) und gezählt wie vorstehend beschrieben.

Etwa 10⁸ Milzzellen wurden mit 2 x 10⁷ SP2/0 Myelomzellen aus der Maus gemischt. Nach 10-minütiger Zentrifugation bei 1000 Upm. wurde der Überstand entfernt und 1 ml Polyethylenglycol (PEG 4000, Firma Merck, 50%) in das Gefäß mit dem Pellet gegeben. Das Pellet wurde dann unter leichtem Klopfen resuspendiert und 1 Minute bei 37°C inkubiert.

10 ml serumfreies DMEM wurden tropfenweise unter leichtem Klopfen zugegeben und das Gemisch 2 bis 4 Minuten inkubiert. Die fusionierten Zellen wurden anschließend 10 Minuten bei 1000 Upm. zentrifugiert. Das erhaltene Zellpellet wurde in 20% fötalem Kälberserum (FCS) und HAT (Hypoxanthin 0,1 µM; Äminopterin 0,4 µM; Thymidin 16 µM) enthaltenem DMEM suspendiert und auf Kulturplatten (Nunc) mit 24 Vertiefungen mit einer näherungsweisen Konzentration von 5 x 10⁴ - 10⁶ Zellen pro Vertiefung ausplattiert. Nach 2 bis 3 Wochen wurden einzelne Zellkolonien aus den einzelnen Vertiefungen entnommen und in Vertiefungen einer neuen Kulturplatte kultiviert.

Die Kulturüberstände wurden auf antigenspezifische Antikörper mittels der EIA-Technik abgesucht. Jede Vertiefung einer mit Acridiniumacylsulfonamid-BSA (3 µ g/ml) überzogenen Mikrotitrationsplatte wurde mit 100 µl des Überstands gefüllt und 1 Stunde bei Raumtemperatur inkubiert. Nach dem Waschen wurden 100 µl eines Anti-Maus-Peroxidase (POD)-Konjugats aus dem Kaninchen für eine weitere Stunde bei Raumtemperatur zugegeben. Nach 30 Minuten Inkubation mit dem Substrat wurde die Farbentwicklung bei 492 nm auf einem Behring-ELISA-Prozessor (BEP) abgelesen. Hybridome, die Antikörper mit einer geeigneten Antigenspezifität herstellen, wurden ausgewählt und unter Verwendung eines Einzelzellmanipulators kloniert. Zur Herstellung großer Mengen monoklonaler Antikörper wurden die Klone in Massenkultur vermehrt. Die anschließende Reinigung der einzelnen monoklonalen Antikörper wurde mittels Protein A-Chromatographie durchgeführt.

### Beispiel 2: Herstellung einer Gensonde (Abb. 3)

Die Synthese des Oligonukleotids ist in der EP-A 0 602 524, S. 49, Beispiel 14 b) beschrieben. Die Kopplung mit dem Acridiniumacylsulfonamid erfolgte nach bekannten Verfahren, die beispielsweise in der obengenannten Europäischen Patentanmeldung beschrieben sind.

### Beispiel 3: Homogener Gensondentest zum Nachweis auf E.coli ohne Anti-Label-MAK

50 µl Standard (aus Flash Track^{®}-Test der Fa. Gen Probe, Lot 11276/11278 für positiven/ negativen Standard) werden in Polystyrolröhrchen pipettiert. Es werden 50 µl der Gensonde gemäß Abb. 3 (2,5 x 10⁶ RLU, 1 M Tris-Puffer, pH7) zugefügt und 15 Minuten bei 60°C hybridisiert. Anschließend werden 300 µl eines Selektionsreagenzes (0.2 M Tetraborat, pH 8) zugegeben, 2 x 3 Sekunden geschüttelt und wiederum 15 Minuten bei 60°C inkubiert. Daraufhin läßt man die Röhrchen 5 Minuten abkühlen.
Die Messung erfolgt durch Zugabe von jeweils 300 µl Analyzerreagenz 1 (0.1 M HNO₃, 0.5 % H₂ O₂) und Analyzerreagenz 2 (0.25 M NaOH) in einem Luminometer (AutocliniLumat^{®} der Fa. Berthold). Die Meßzeit beträgt 1 sec/Probe.

Es wird eine deutliche Signaldifferenzierung zwischen positiven und negativem Standard ermittelt (s. Tabelle).

### Beispiel 4: Homogener Gensondentest zum Nachweis auf E.coli mit Anti-Label-MAK

50 µl Standard (aus Flash Track^{®}-Test der Fa. Gen Probe, Lot 11276/11278 für positiven/ negativen Standard) werden in Polystyrolröhrchen pipettiert. Es werden 50 µl der Gensonde gemäß Abb. 3 (2,5 x 10⁶ RLU, 1 M Tris-Puffer, pH7) zugefügt und 15 Minuten bei 60°C hybridisiert. Anschließend werden 300 µl eines Selektionsreagenzes (0.2 M Tetraborat, pH8) zugegeben, 2 x 3 Sekunden geschüttelt und wiederum 15 Minuten bei 60°C inkubiert. Daraufhin läßt man die Röhrchen 5 Minuten abkühlen.

Anschließend werden 50 µl Anti-Label-MAK-Lösung (10 µg/ml Tris-Puffer pH7.4, 1 M, 0.1 % Triton X-100) 1 Minute bei RT inkubiert.
Die Messung erfolgt durch Zugabe von jeweils 300 µl Analyzerreagenz 1 (0.1 M HNO₃, 0.5 % H₂ O₂) und Analyzerreagenz 2 (0.25 M NaOH) in einem Luminometer (AutoCliniLumat^{®} der Fa. Berthold). Die Meßzeit beträgt 1 sec/Probe.

Im Vergleich zum ursprünglichen "hybridization protection assay" (Beispiel 3) ist die Signaldifferenzierung zwischen positivem und negativem Standard durch Zugabe des Anti-Label-MAK deutlich verbessert (s. Tabelle). Es wird eine Signaldifferenzierung erreicht, die ansonsten nur in einer heterogenen Ausführungsform mit Magnetpartikeln als Festphase möglich ist (s. EP-A-0 602 524, S. 50 bis 51, Beispiel 17).

### Beispiel 5: Homogener Gensondentest zum Nachweis auf E.coli mit unspezifischem MAK (Kontrollexperiment)

50 µl Standard (aus Flash Track^{®}-Test der Fa. Gen Probe, Lot 11276/11278 für positiven/ negativen Standard) werden in Polystyrolröhrchen pipettiert. Es werden 50 µl der Gensonde gemäß Abb. 3(2,5 x 10⁶ RLU, 1 M Tris-Puffer, pH7) zugefügt und 15 Minuten bei 60°C hybridisiert. Anschließend werden 300 µl eines Selektionsreagenzes (0.2 M Tetraborat, pH8) zugegeben, 2 x 3 Sekunden geschüttelt und wiederum 15 Minuten bei 60°C inkubiert. Daraufhin läßt man die Röhrchen 5 Minuten abkühlen.

Anschließend werden 50 µl Anti-TSH-MAK-Lösung (1 µg TSH-MAK der Fa. Medix, Ch.-Nr.:SPHY052/ml Tris-Puffer pH 7.4, 1 M, 0.1 % Triton X-100) 5 Minuten bei RT inkubiert.
Die Messung erfolgt durch Zugabe von jeweils 300 µl Analyzerreagenz 1 (0.1 M HNO₃, 0.5 % H₂ O₂) und Analyzerreagenz 2 (0.25 M NaOH) in einem Luminometer (AutoCliniLumat^{®} der Fa. Berthold).
Die Meßzeit beträgt 1 sec/Probe.

Die Signaldifferenzierung zwischen positivem und negativem Standard entspricht - wie erwartet - dem Beispiel 3 (s. Tabelle).

**Tabelle:**

| | **Beispiel 3** | **Beispiel 4** | **Beispiel 5** |
|---|---|---|---|
| **Positiver Standard** | 13449 | 14229 | 13872 |
| **[RLU]** | 14066 | 14041 | 14119 |
| **Negativer Standard** | 416 | 53 | 408 |
| **[RLU]** | 404 | 47 | 419 |

| | | | |
|---|---|---|---|
| (RLU = "relative light units") | | | |

## Patentansprüche

1. Verfahren zur Bestimmung einer Targetsequenz, worin eine die Targetsequenz gegebenenfalls enthaltende Probe, mit einer zur Bestimmung dieser Targetsequenz geeigneten markierten Gensonde so in Kontakt gebracht wird, daß die Targetsequenz und die markierte Gensonde miteinander hybridisieren, **dadurch gekennzeichnet, daß** zur Verminderung des Störeinflusses durch die nicht hybridisierte markierte Gensonde
(a) ein Rezeptor zugesetzt wird, der an das Label einer gegebenenfalls überschüssigen, nicht an die Targetsequenz hybridisierten Fraktion der Gensonde bindet, wodurch das auf das Label zurückzuführende Signal qualitativ und/oder quantitativ verändert wird und
(b) das auf das Label zurückzuführende Signal mit einem dafür geeigneten Verfahren qualitativ oder quantitativ detektiert wird.

2. Verfahren nach Anspruch 1, **dadurch gekennzeichnet, daß** in einem zusätzlichen Schritt das Label einer gegebenenfalls überschüssigen, nicht hybridisierten Gensonde durch Inkubation mit einem Selektionsreagenz, welches eine selektive Hydrolyse der einzelsträngigen Gensonde bewirkt, teilweise inaktiviert wird.

3. Verfahren nach Anspruch 2, **dadurch gekennzeichnet, daß** zuerst die Inkubation mit dem Selektionsreagenz erfolgt und anschließend die Zugabe des Rezeptors.

4. Verfahren nach einem oder mehreren der vorhergehenden Ansprüche, **dadurch gekennzeichnet, daß** der Rezeptor ein monoklonaler oder polyklonaler Antikörper, ein Antikörperfragment, ein chemisch modifizierter Antikörper oder ein chemisch modifiziertes Antikörperfragment ist, sofern die Antigenbindungsfähigkeit nach der chemischen Modifikation in ausreichendem Maße erhalten bleibt.

5. Verfahren nach einem oder mehreren der vorhergehenden Ansprüche, **dadurch gekennzeichnet, daß** das Label eine zur Fluoreszenz, Phosphoreszenz, Chemilumineszenz, Biolumineszenz oder Elektrolumineszenz befähigte Gruppe ist.

6. Verfahren nach Anspruch 5, **dadurch gekennzeichnet, daß** das Label ein Acridiniumester, ein Acridiniumacylsulfonamid, ein Luminol, ein Isoluminol oder ein Derivat davon, ein Dioxetan, ein Luciferin, ein Oxalsäureester oder ein Oxalsäureamid ist.

7. Verfahren nach einem oder mehreren der Ansprüche 1 bis 4, **dadurch gekennzeichnet, daß** das Label ein Enzym ist.

## Claims

1. A method for the determination of a target sequence, in which a sample optionally containing the target sequence is brought into contact with a labeled gene probe suitable for the determination of this target sequence such that the target sequence and the labeled gene probe hybridize with one another, which comprises, to reduce the interfering effect due to the nonhybridized labeled gene probe,
(a) adding a receptor which binds to the label of an optionally excess fraction of the gene probe not hybridized to the target sequence, whereby the signal to be attributed to the label is qualitatively and/or quantitatively altered and
(b) qualitatively or quantitatively detecting the signal to be attributed to the label using a method suitable for this purpose.

2. The method as claimed in claim 1, wherein, in an additional step, the label of an optionally excess, nonhybridized gene probe is partly inactivated by incubation with a selection reagent which causes a selective hydrolysis of the single-stranded gene probe.

3. The method as claimed in claim 2, wherein first the incubation with the selection reagent takes place and then the addition of the receptor.

4. The method as claimed in one or more of the preceding claims, wherein the receptor is a monoclonal or polyclonal antibody, an antibody fragment, a chemically modified antibody or a chemically modified antibody fragment, if the antigen-binding capacity after the chemical modification is retained to an adequate extent.

5. The method as claimed in one or more of the preceding claims, wherein the label is a group capable of fluorescence, phosphorescence, chemiluminescence, bioluminescence or electroluminescence.

6. The method as claimed in claim 5, wherein the label is an acridinium ester, an acridinium acylsulfonamide, a luminol, an isoluminol or a derivative thereof, a dioxetane, a luciferin, an oxalic acid ester or an oxamide.

7. The method as claimed in one or more of claims 1 to 4, wherein the label is an enzyme.

## Revendications

1. Procédé pour la détermination d'une séquence cible, dans lequel on met un échantillon, contenant éventuellement la séquence cible, en contact avec une sonde génique marquée, appropriée à la détermination de cette séquence cible, de manière que la séquence cible et la sonde génique marquée s'hybrident l'une avec l'autre, **caractérisé en ce que**, pour diminuer l'influence perturbatrice de la sonde génique marquée non hybridée,
(a) on ajoute un récepteur qui se lie au marqueur d'une fraction, éventuellement en excès, non hybridée avec la séquence cible, de la sonde génique, de sorte que le signal à attribuer au marqueur est modifié qualitativement et/ou quantitativement et
(b) on détecte qualitativement ou quantitativement par un procédé approprié à cette fin le signal à attribuer au marqueur.

2. Procédé selon la revendication 1, **caractérisé en ce que** dans une étape supplémentaire le marqueur d'une sonde génique non hybridée, éventuellement en excès, est partiellement inactivé par incubation avec un réactif de sélection qui provoque une hydrolyse sélective de la sonde génique simple brin.

3. Procédé selon la revendication 2, **caractérisé en ce qu'**on effectue d'abord l'incubation avec le réactif de sélection et ensuite l'addition du récepteur.

4. Procédé selon une ou plusieurs des revendications précédentes, **caractérisé en ce que** le récepteur est un anticorps monoclonal ou polyclonal, un fragment d'anticorps, un anticorps modifié chimiquement ou un fragment d'anticorps modifié chimiquement, dans la mesure où l'aptitude à la fixation à l'antigène est maintenue à un degré suffisant après la modification chimique.

5. Procédé selon une ou plusieurs des revendications précédentes, **caractérisé en ce que** le marqueur est un groupe apte à la fluorescence, à la phosphorescence, à la chimiluminescence, à la bioluminescence ou à l'électroluminescence.

6. Procédé selon la revendication 5, **caractérisé en ce que** le marqueur est un ester d'acridinium, un acylsulfonamidure d'acridinium, un luminol, un isoluminol ou un dérivé de ceux-ci, un dioxétanne, une luciférine, un ester d'acide oxalique ou un amide d'acide oxalique.

7. Procédé selon une ou plusieurs des revendications 1 à 4, **caractérisé en ce que** le marqueur est une enzyme.
